(19) Europäisches Patentamt
European Patent Office
Office européen des brevets

(11) **EP 1 319 681 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**19.09.2012 Patentblatt 2012/38**

(51) Int Cl.:
*C08J 3/12* (2006.01)          *C08G 69/46* (2006.01)

(45) Hinweis auf die Patenterteilung:
**20.12.2006 Patentblatt 2006/51**

(21) Anmeldenummer: **02023443.1**

(22) Anmeldetag: **19.10.2002**

(54) **Verfahren zur Herstellung eines PH-Wert-geregelten Polyamidpulver**

Process for the preparation of a PH controlled polyamide powder

Procédé de préparation d'une poudre de polyamide à pH controlé

(84) Benannte Vertragsstaaten:
**AT BE BG CH CY CZ DE DK EE ES FI FR GB GR IE IT LI LU MC NL PT SE SK TR**

(30) Priorität: **12.12.2001 DE 10161038**

(43) Veröffentlichungstag der Anmeldung:
**18.06.2003 Patentblatt 2003/25**

(73) Patentinhaber: **Evonik Degussa GmbH**
**45128 Essen (DE)**

(72) Erfinder:
• **Schiffer, Thomas, Dr.**
  **45721 Haltern (DE)**
• **Renners, Holger**
  **46342 Velen (DE)**
• **Christoph, Wolfgang**
  **45768 Marl (DE)**
• **Baumann, Franz-Erich, Dr.**
  **48249 Dülmen (DE)**
• **Mügge, Joachim, Dr.**
  **45721 Haltern (DE)**

(56) Entgegenhaltungen:
**FR-A- 2 365 597     US-A- 3 679 638**
**US-A- 4 069 184**

EP 1 319 681 B2

**Beschreibung**

Die Erfindung betrifft ein Verfahren zur Herstellung von Polyamidpulver für Kosmetikanwendungen, welches in wässriger Suspension einen definierten pH-Wert aufweist, sowie dessen Herstellung. Bevorzugt wird ein pH-Wert eingestellt, der im Bereich des natürlichen Säureschutzmantels der Haut liegt.

Dies wird erfindungsgemäß durch Zugabe eines Puffersystems erreicht, welches auf Basis natürlicher, das heißt im Körper des Menschen vorkommender organischer und/oder mineralischer Säuren und deren Salzen besteht. Typische Vertreter dafür sind Phosphatpuffer und/oder Citratpuffer.

Das erfindungsgemäße hergestellte Kosmetikpulver besitzt eine mittlere Kornverteilung ($d_{50}$) zwischen 1 und 400 $\mu$m, bevorzugt 5 bis 100, besonders bevorzugt 5 bis 60.

Die Herstellung der Kosmetikpulver kann als Dry Blend erfolgen. Hierbei werden Polyamidpulver und Puffersystem als Feststoffe eingesetzt. Das Puffersystem kann sowohl zuerst alleine vermahlen und anschließend mit dem Polyamidfeinpulver gemischt werden. Oder es werden Puffersystem und Polyamidpulver zunächst miteinander vermischt und anschließend gemeinsam nachgemahlen und gesichtet.

Ein weiteres erfindungsgemäßes Herstellverfahren besteht darin, dass eine Pufferlösung auf das Polyamidpulver gegeben und anschließend gemeinsam mit dem Polyamid vom Lösemittel befreit wird. Hierbei gelingt eine besonders gleichmäßige Verteilung des Puffersystems im Polyamidkorn, welche sich insbesondere für poröse Fällpulver eignet. Das Verfahren wird bevorzugt in den Trocknungsschritt des Fällprozesses integriert, kann aber auch nachträglich an einem trockenen Feinpulver erfolgen.

Polyamidfeinpulver finden Anwendung in zahlreichen Kosmetikprodukten, wie zum Beispiel in Lippenstiften, Make-ups, Pudern und Cremes. Sie enthalten in der Regel Feinpulver auf Basis Polyamid 11 und/oder Polyamid 12, sowie Polyamid 6 und PA6/12 Copolymere mit einem mittleren Korndurchmesser von 5 bis 30 $\mu$m. Polyamidpulver mit einem größeren mittleren Korndurchmesser von bis zu 400 $\mu$m finden unter anderem Anwendung in Duschgels, um einen sogenannten Peelingeffekt zu erzielen.

Da Kosmetika in direktem Kontakt mit der menschlichen Haut stehen, werden hohe dermatologische Anforderungen an ihre Inhaltsstoffe bezüglich ihrer Reinheit und ihrer Hautverträglichkeit gestellt. Bisherige Polyamidfeinpulver erfüllen die hohen Reinheitsanforderungen bereits. Bekannt zur Herstellung solcher Kosmetikpulver auf Basis Polyamid sind vor allem das Fällverfahren und die anionische Polymerisation. Andere spezielle Verfahren zur Herstellung von Kosmetikpulvern, wie in JP 04050232 und JP 05070598 (beide Daicel-Hüls) und JP 05025019 (Kao Corp.) erwähnt, haben keine technische Bedeutung erlangt.

Pulver aus beiden Verfahren weisen jedoch häufig aufgrund ihres jeweiligen Herstellungsverfahrens pH-Werte auf, die außerhalb des Bereiches liegen, der von Dermatologen als der optimale pH-Bereich für den Säureschutzmantel der Haut angesehen wird. Sowohl zu saure wie zu basische pH-Werte können zu Hautreizungen führen. Insbesondere basische pH-Werte fördern zudem das Austrocknen der Haut. Dadurch wird die Haut spröde, ihre Elastizität nimmt ab. Bei Beanspruchung kann es schnell zu Rissen und kleinen Hautverletzungen kommen.

Das Fällverfahren wird in DE 29 06 647 B1 beschrieben. Hierbei wird ein geeignetes Polyamid-12 Granulat unter Druck in heißem Alkohol vollständig gelöst. Beim Abkühlen kristallisieren feine Partikel aus, deren Wachstum durch die Abkühlrate und gegebenenfalls auch durch geringe Mengen an phosphorhaltigen Säuren gesteuert wird. Nach abgeschlossener Kristallisation der Polyamidpartikel wird der Alkohol abgezogen. Geringe Mengen an phosphorhaltigen Säuren (z. B. Hypophosphorige Säure, Phosphorige Säure und/oder Phosphorsäure) bleiben jedoch im Polyamid eingeschlossen, wodurch diese Feinpulver in wässriger Suspension tendenziell leicht sauer reagieren je nach Carboxylendgruppenüberschuss und Phosphorsäuregehalt liegen die pH-Werte bei 6 - 4, in Extremfällen sogar unter pH 4. Diese pH-Werte entsprechen auf bestimmten Gebieten nicht den Marktanforderungen.

Bei der anionischen Lösungspolymerisation findet eine anionisch aktivierte Polymerisation von monomerem Lactam, vorzugsweise Laurinlactam, in höhersiedenden Paraffinen (140 bis 170 °C) statt. Dafür werden stark basische Katalysatoren verwendet, weshalb solche Feinpulver trotz mehrfacher Wäsche in der Regel leicht basisch / alkalisch reagieren. Die pH-Werte solcher Pulver liegen zwischen 7 und 8,5.

Die aus beiden Verfahren erhaltenen Feinpulver zeigen eine runde Partikelform. Pulver aus beiden Verfahren werden in der Regel schutzgesiebt und/oder gesichtet und können gegebenenfalls zur Erhöhung des Feinanteils nachgemahlen werden.

In den meisten Kosmetikzubereitungen sind in der Regel nur zwischen 0,1 und 10 Gew.-% Polyamidfeinpulver enthalten. Je nach Anwendungsbereich besteht der Rest aus diversen Ölen, Fetten, Emulgatoren, Stabilisatoren, sowie Farb- und Aromastoffen. Sie enthalten in der Regel ein ausreichend großes Puffersystem, um eine Kosmetikanwendung mit einem hautfreundlichen pH-Wert zu erzeugen.

Jedoch besteht seit einiger Zeit von Seiten der Kosmetikindustrie der explizite Wunsch auch nach pH-Wert geregelten Polyamidpulvern.

Überraschend wurde jetzt gefunden, dass sich Polyamidpulver, wie in den Ansprüchen beschrieben, insbesondere Fällpulver, Polyamid-Pulver die nach dem Fällverfahren hergestellt wurden, gut im pH-Wert regeln lassen, wenn sie mit einem speziellen Puffersystem ausgestattet werden. Dieses Puffersystem beruht dabei auf natürlichen organischen und/oder mineralischen Säuren und ihren entsprechenden Salzen. Prinzipiell kann auch das leicht basische Polyamidpulver mit dem erfindungsgemäßen Verfahren abgepuffert werden.

Als Polyamidpulver eignen sich insbesondere Pulver, die Polyamid 11 und/oder Polyamid 12 aufweisen. Besonders bevorzugt wird ein Polyamid 12 Fällpulver mit porösen Oberflächen, wie sie nach DE 29 06 647 B 1 entstehen.

Als Puffersystem wird die Kombinationen schwacher organischer und/oder mineralischer Säuren und ihrer korrespondierenden Salze verstanden.

Unter natürlich vorkommenden Säuren werden organische und/oder mineralische Verbindungen verstanden, die entweder im menschlichen Körper selbst gebildet werden oder vom Menschen regelmäßig, beispielsweise mit der Nahrung oder über die Haut aufgenommen werden können, ohne dass es dadurch bekanntermaßen zu gesundheitlichen Beeinträchtigungen kommt. Wichtig ist hierbei auch, dass die Verbindungen vom menschlichen Organismus vollständig abgebaut werden können und dass bekanntermaßen weder die aufgenommenen Verbindungen selbst noch ihre Metabolite zu gesundheitlichen Schäden führen.

Als Salze können alle korrespondierenden Basen zu den oben genannten Säuren verwendet werden. In der Regel werden dafür physiologisch unbedenkliche Alkali- und Erdalkalisalze, sowie Ammoniumsalze verwendet, doch sind auch andere Kationen möglich.

Der Begriff der Säure umfasst im Sinne der Erfindung auch die entsprechenden Hydrogensalze mehrprotoniger Säuren, zum Beispiel Natriumdihydrogenphosphat oder Dinatriumhydrogencitrat.

Von Dermatologen wird nach dem derzeitigen Stand der Erkenntnis für Anwendungen auf die Haut allgemein ein pH-Bereich zwischen 4 und 7 empfohlen, um Reizungen oder Austrocknung der Haut zu vermeiden. Erfindungsgemäß wird der pH-Bereich zwischen 4,5 und 6,5, ganz besonders zwischen 5,0 und 6,0 eingestellt.

Ein konkreter "optimaler" pH-Wert der Haut lässt sich nicht allgemein angeben. Er unterscheidet sich bei jedem Menschen etwas aufgrund des jeweiligen Hauttyps. Auch hängt er vom Geschlecht und dem Alter des jeweiligen Menschen ab (siehe u.a. Römpp Chemielexikon, 9. Auflage, S. 1743).

Voraussetzung für den Einsatz der jeweiligen Säure und ihrer korrespondierenden Basen ist, dass das daraus resultierende System einen Puffer ausreichender Kapazität im oben genannten pH-Bereich ausbildet. Entscheidende Kenngröße bei der Auswahl der Säure ist dabei der pKs-Wert. Aus diesem lässt sich über die sogenannte Henderson-Hasselbalch-Gleichung der jeweilige pH-Wert einer wässrigen Lösung ermitteln:

$$pH = pKs - \log \{c(HA) / c(A^-)\}$$

mit HA = Säure und $A^-$ = korrespondierende Base

Bei mehrprotonigen Säuren werden anstelle der freien Säure die entsprechenden Mono- oder Dihydrogensalze verwendet. Einige pKs-Werte bei 25 °C geeigneter dreiprotoniger Säuren für Puffersysteme sind als nicht limitierende Beispiele im Folgenden angegeben. Dabei sind die für die Kosmetikpulver geeigneten pKs-Werte hervorgehoben:

Zitronensäure:
$pKs_1$= 3.128, $pKs_2$= 4,761, **$pKs_3$= 6,396**

Phosphorsäure
$pKs_1$= 2,15, **$pKs_2$= 7,09,** $pKs_3$= 12,32

Für die Zitronensäure eignet sich als Puffer der $pKs_3$-Wert, also zum Beispiel das System Dinatriumhydrogencitrat / Trinatriumcitrat. Für die Phosphorsäure ist es der $pKs_2$-Wert, zum Beispiel in Form des Systems Natriumdihydrogenphosphat / Dinatriumhydrogenphosphat.

Der Gehalt an Puffersystem im Kosmetikpulver sollte möglichst gering gehalten werden. Die kosmetischen Eigenschaften des Polyamidpulvers sollten auch durch den Gehalt an Puffersystem möglichst wenig beeinflußt werden. Gleichzeitig soll aber eine ausreichende Pufferkapazität erreicht werden, um den gewünschten pH-Wert im Kosmetikpulver sicher halten zu können.

Bei der Zusammenstellung der Puffermischung ist es vorteilhaft, den Gehalt an Säure- und Basefunktionen im Polyamid mit einzubeziehen. Dies betrifft sowohl die Carboxyl- und Aminoendgruppen der Polyamidketten, als auch saure oder basische Zuschlagstoffe, die während der Polymerisation als Katalysator (z. B. phosphorhaltige Säure) oder als Regler (z.B. Dodekandisäure) fungiert haben.

Der Gehalt an Säure- und Aminofunktionen im Polyamid lässt sich z. B. durch titrimetrische Methoden leicht quantifizieren. Diese sind allgemein bekannt und können zum Beispiel im Kunststoff-Handbuch, Band 3,4 "Polyamide", Hanser Verlag, München nachgelesen werden.

Das Puffersystem, umfassend Säure und korrespondierende Base, wird so gewählt, dass 0,01 bis 2 Teile des Puffers auf 100 Teile Polyamid kommen und besonders bevorzugt 0,1 bis 1 Teil.

Die Einarbeitung des Puffersystem ins Polyamid kann dabei erfindungsgemäß über verschiedene Verfahren erfolgen.

Beim sogenannten Dry-Blend werden Puffersystem und Polyamidpulver trocken und intensiv vermischt. Voraussetzung dafür ist, dass das gemahlene Puffersystem keinen deutlich größeren mittleren Partikeldurchmesser aufweist als das jeweilige Polyamidfeinpulver. Bevorzugt wird das Puffersystem zunächst trocken feinst vermahlen, so dass der mittlere Korndurchmesser deutlich kleiner ist als der mittlere Durchmesser der Polyamidpartikel. Das Puffersystem kann anschließend beim Mischen in die Poren des Polyamidkorns eindringen. Besonders geeignet dafür sind poröse Polyamid-Fällpulver.

Zur Herstellung von Polyamid-Feinpulvern mit einem mittleren Partikeldurchmesser $d_{50}$ von unter 20 $\mu$m empfiehlt es sich, dass Polyamidpulver und das Puffersystem zunächst miteinander vermischt werden und anschließend in einem gemeinsamen Schritt auf die gewünschte Partikelgröße nachgemahlen und gesichtet werden.

Alternativ zum Dry Blend lässt sich das Puffersystem auch in Lösung auf die Polyamidpartikel aufziehen. Dieses Verfahren ist prinzipiell bei allen Polyamidpulvern anwendbar. Bei einem Fällpulver wie VESTOSINT ist dieses Verfahren jedoch besonders vorteilhaft. Dieser Schritt kann in diesem Fall auch bereits in einem kombinierten Mischer/Trockner direkt in den Fällprozess integriert werden. Dadurch lassen sich Zeit und ein Arbeitsschritt einsparen, wenn die Trocknung des Polyamidpulvers aus dem Fällprozess und Einarbeitung des Puffersystems im gleichen Behälter erfolgen.

Prinzipiell wird bei diesem Verfahren das Puffersystem in einem Lösemittel gelöst auf die Polyamidpartikel aufgesprüht. Als Lösemittel eignen sich bevorzugt Wasser und/oder wässrig alkoholische Lösungen. Anschließend wird das Lösemittel bei erhöhten Temperaturen im Mischer, bzw unter Vakuum, vollständig abgezogen.

Die erhöhten Temperaturen im Mischer bewirken, dass bei diesem Verfahren das Puffersystem sehr fein und gleichmäßig auf dem Polyamidkorn verteilt vorliegt, und dass freie Carboxyl- und Aminoendgruppen im Polyamid bereits chemisch reagieren und vollständig abgepuffert werden.

Die Bestimmung des pH-Wertes der Polyamidpulver erfolgt jeweils in Suspension von 1 g Pulver in 100 ml destilliertem Wasser. Nach 24 h Rühren sind die eingeschlossenen Säuren und/oder Basen aus dem Polyamid herausgelöst. Der pH-Wert wird über eine kalibrierte pH-Elektrode bestimmt. Messwerte sind in Tabelle 1 zusammengestellt.

Beispiel 1 Zur Herstellung der Polyamidfeinpulver, Fällverfahren entsprechend DE 29 06 647 B1:

60 kg ungeregeltes, durch hydrolytische Polymerisation von Laurinlactam hergestelltes PA12 mit einer relativen Lösungsviskosität $\eta$rel von 1.61 (in angesäuertem m-Kresol) und einem Endgruppengehalt von 72 mmol/kg COOH und 68 mmol/kg $NH_2$ werden mit 375 1 Ethanol, vergällt mit 2-Butanon und 1 % Wassergehalt in einem Rührkessel auf 145 °C gebracht und unter Rühren (Blattrührer) 1 Stunde bei dieser Temperatur belassen.

Anschließend wird die Manteltemperatur auf 124 °C reduziert, 60 g Phosphorsäure als Kristallisationshilfe zugegeben und unter kontinuierlichem Abdestillieren des Ethanols mit einer Kühlrate von 25 K/h unter Rühren die Innentemperatur auf 125 °C gebracht. Von jetzt an wird bei gleicher Kühlrate die Manteltemperatur 2 K - 3 K unter der Innentemperatur gehalten, bis bei 109 °C die Fällung, erkennbar an der Wärmeentwicklung, einsetzt. Durch Abdestillieren von Ethanol wird erreicht, dass die Innentemperatur nicht über 109.5 °C ansteigt. Nach ca. 20 Minuten fällt die Innentemperatur ab, was das Ende der Fällung anzeigt. Durch weiteres Abdestillieren und Kühlung über den Mantel wird die Temperatur der Suspension auf 45 °C gebracht und die Suspension danach in einen Trockner überführt. Das Ethanol wird bei 70 °C/400 mbar abdestilliert, und der Rückstand anschließend bei 20 mbar/85 °C 3 Stunden nachgetrocknet.

Die gewünschten Feinfraktionen werden durch mehrfaches Sieben und Sichten entsprechend ihrem mittleren Korndurchmesser abgetrennt.

Beispiel 2 Zur Herstellung der Polyamidfeinpulver, Fällverfahren, anionische Polymerisation entsprechend EP 0 192 515:

In einem Rundkolben werden unter Schutzgas (Argon) 300 ml trockenes Decalin vorgelegt. In der Wärme (80 °C) werden unter Rühren (600 U/min) 80 g Laurinlactam und 3 g N,N'-Ethylendiamin-bis-oleamid gelöst und 400 mg Siliziumdioxid als Keimbildner zugesetzt. Spuren von Wasser werden aus der Reaktionsmischung entfernt, indem ca. 10 % des Lösemittels im Vakuum bei 120 bis 130 °C abdestilliert werden. Unter Schutzgas werden anschließend bei ca. 110 °C und Normaldruck 175 mg Natriumhydrid zugesetzt (Firma Fluka, ca. 60 %ig in Öl). Man hält die Reaktionsmischung noch 30 Minuten bei 110 °C, kühlt anschließend mit 0,2 K/min auf 100 °C ab. Über eine Dosierpumpe werden innerhalb 2 h 4,5 g Stearylisocyanat zugegeben. Nach Abkühlen auf 90 °C wird der ausgefallene Niederschlag als feines weißes Pulver abgetrennt. Er wird mit 100 ml Ethanol bei Raumtemperatur gewaschen und im Vakuumtrockenschrank bei 200 mbar, 60 °C getrocknet.

Beispiel 3 (Vergleichsbeispiel):

1,0 g des Polyamid 12-Fällpulvers aus Beispiel 1 mit einer mittleren Korngröße $d_{50}$ = 20 $\mu$m werden in 100 ml destilliertem Wasser suspendiert und 24 h gerührt. Der pH-Wert wird über ein Digital pH-Meter (Firma Schott CG 843) gemessen. Die Ergebnisse sind in Tabelle 1 zusammengefasst.

Beispiel 4 (Vergleichsbeispiel):

Analog zu Beispiel 3 werden 1,0 g anionisch polymerisiertes Polyamid 12-Pulver aus Beispiel 2 in 100 ml destilliertem Wasser suspendiert und 24 h gerührt. Der pH-Wert wird über ein Digital pH-Meter (Firma Schott CG 843) gemessen. Die Ergebnisse sind in Tabelle 1 zusammengefasst.

Beispiel 5: (nicht erfindungsgemäß)

100 g des Polyamid 12-Fällpulvers aus Beispiel 1 mit einer mittleren Korngröße $d_{50}$ = 20 $\mu$m wird mit 0,1 g fein gemahlenem und gesichtetem Trinatriumcitrat Dihydrat ($d_{50} \leq$ 10 $\mu$m) versetzt und im Dry-Blend auf einem Diosna-Mischer 3 Minuten bei 500 U/Min vermischt. Von der Mischung wird 1,0 g in 100 ml destilliertem Wasser suspendiert und 24 h gerührt. Der pH-Wert wird über ein Digital pH-Meter (Firma Schott CG 843) gemessen. Die Ergebnisse sind in Tabelle 1 zusammengefasst.

Beispiel 6: (nicht erfindungsgemäß)

Der Versuch von Beispiel 5 wird mit 0,5 g fein gemahlenem und gesichtetem Trinatriumcitrat Dihydrat ($d_{50} \leq$ 10 $\mu$m) wiederholt. Die Ergebnisse sind in Tabelle 1 zusammengefasst.

Beispiel 7:

Der Versuch von Beispiel 5 wird mit 1,2 g fein gemahlenem und gesichtetem Trinatriumcitrat Dihydrat ($d_{50} \leq$ 10 $\mu$m) und 0,5 g fein gemahlenem und gesichtetem Dinatriumhydrogencitrat Sesquihydrat ($d_{50} \leq$ 10 $\mu$m) wiederholt. Die Ergebnisse sind in Tabelle 1 zusammengefasst.

Beispiel 8:

Der Versuch von Beispiel 5 wird mit 0,5 g fein gemahlenem und gesichtetem Trinatriumcitrat Dihydrat ($d_{50} \leq$ 10 $\mu$m) und 1,2 g fein gemahlenem und gesichtetem Dinatriumhydrogencitrat Sesquihydrat ($d_{50} \leq$ 10 $\mu$m) wiederholt. Die Ergebnisse sind in Tabelle 1 zusammengefasst.

Beispiel 9: (nicht erfindungsgemäß)

Der Versuch von Beispiel 5 wird mit 2,0 g fein gemahlenem und gesichtetem Trinatriumcitrat Dihydrat ($d_{50} \leq$ 10 $\mu$m) wiederholt. Die Ergebnisse sind in Tabelle 1 zusammengefasst.

Beispiel 10: (nicht erfindungsgemäß)

60 kg Fällpulver wurden entsprechend dem Versuch von Beispiel 1 hergestellt und im Mischer bei 70 °C/400 mbar 30 min vorgetrocknet. Das noch feuchte Pulver wird unter Rühren mit einer Lösung von 960 g Trinatriumcitrat Dihydrat und 5 l destilliertem Wasser besprüht. Die Lösemittel werden bei 70 °C/400 mbar abdestilliert und der Rückstand anschließend bei 20 mbar/85 °C 3 Stunden nachgetrocknet.
Die gewünschten Feinfraktionen werden durch mehrfaches Sieben und Sichten entsprechend ihrem mittleren Korndurchmesser abgetrennt.

Beispiel 11: (nicht erfindungsgemäß)

1,00 g des Polyamid 12-Fällpulvers aus Beispiel 10 mit einer mittleren Korngröße $d_{50}$ = 20 $\mu$m werden in 100 ml destilliertem Wasser suspendiert und 24 h gerührt. Der pH-Wert wird über ein Digital pH-Meter (Firma Schott CG 843) gemessen. Die Ergebnisse sind in Tabelle 1 zusammengefasst.

Beispiel 12:

60 kg Fällpulver wurden entsprechend dem Versuch von Beispiel 1 hergestellt und im Mischer bei 70 °C/400 mbar 30 min vorgetrocknet. Das noch feuchte Pulver wird unter Rühren mit einer Lösung von 720 g Trinatriumcitrat Dihydrat, 300 g Dinatriumhydrogencitrat Sesquihydrat und 5 1 destilliertem Wasser besprüht. Die Lösemittel werden bei 70 °C/400 mbar abdestilliert und der Rückstand anschließend bei 20 mbar/85 °C 3 Stunden nachgetrocknet.
Die gewünschten Feinfraktionen werden durch mehrfaches Sieben und Sichten entsprechend ihrem mittleren Korndurchmesser abgetrennt.

Beispiel 13:

1,00 g des Polyamid 12-Fällpulvers aus Beispiel 12 mit einer mittleren Korngröße $d_{50}$ = 20 μm werden in 100 ml destilliertem Wasser suspendiert und 24 h gerührt. Der pH-Wert wird über ein Digital pH-Meter (Firma Schott CG 843) gemessen. Die Ergebnisse sind in Tabelle 1 zusammengefasst.

**Tabelle 1**

| Beispiel | Trinatriumcitrat * Dihydrat [g/100g] | Dinatriumhydrogencitrat * Sesquihydrat [g/100g] | pH-Wert [nach 24 h] |
|---|---|---|---|
| 3 | - | - | 4,6 |
| 4 | - | - | 8,4 |
| 5 | 0,1 | - | 5,0 |
| 6 | 0,5 | - | 6,2 |
| 7* | 1,2 | 0,5 | 6,4 |
| 8* | 0,5 | 1,2 | 5,9 |
| 9 | 2,0 | - | 7,2 |
| 11 | 1,6 | - | 6,3 |
| 13* | 1,2 | 0,5 | 6,2 |
| * erfindungsgemäß | | | |

**Patentansprüche**

1. Verfahren zur Herstellung eines Polyamidpulvers für Kosmetikanwendungen,
   **dadurch gekennzeichnet,**
   **dass** durch Einarbeitung eines zugefügten Puffersystems aus natürlichen organischen und/oder mineralischen Säuren und ihren korrespondierenden Salzen als Basen in das Polyamidpulver der pH-Wert des Polyamidpulvers zwischen 4,5 und 6,5 eingestellt wird und das Puffersystem in Mengen von 0,01 bis 2 Teile des Puffers auf 100 Teile Polyamid verwendet wird.

2. Verfahren nach Anspruch 1,
   **dadurch gekennzeichnet,**
   **dass** als Puffersystem ein Citrat- und/oder Phosphatpuffer eingesetzt wird.

3. Verfahren nach einem der vorherigen Ansprüche,
   **dadurch gekennzeichnet,**
   **dass** das Pulver einen mittleren Partikeldurchmesser zwischen 1 und 400 μm aufweist.

4. Verfahren nach einem der vorherigen Ansprüche,
   **dadurch gekennzeichnet,**
   **dass** als Polyamid ein Polyamid 11 und/oder Polyamid 12 verwendet wird.

5. Verfahren nach einem der vorhergehenden Ansprüche,
   **dadurch gekennzeichnet,**

**dass** das Polyamid in einem Fällprozess oder durch anionische Lösungspolymerisation hergestellt wurde.

6. Verfahren zur Herstellung eines Polyamidpulvers für Kosmetikanwendungen nach einem der vorherigen Ansprüche, **dadurch gekennzeichnet,** **dass** das Puffersysterm im Dry-Blend eingemischt wird.

7. Verfahren zur Herstellung eines Polyamidpulvers für Kosmetikanwendungen nach einem der vorherigen Ansprüche, **dadurch gekennzeichnet,** **dass** das Puffersystem in Lösung auf das Polyamidpulver gesprüht und anschließend getrocknet wird.

**Claims**

1. Process for the preparation of a polyamide powder for cosmetic applications, **characterized in that** the pH of the polyamide powder is set between 4.5 and 6.5 by incorporating an added buffer system of natural organic and/or mineral acids and their corresponding salts as bases into the polyamide powder and the buffer system is used in amounts of from 0.01 to 2 parts of the buffer per 100 parts of polyamide.

2. Process according to Claim 1 **characterized in that** the buffer system used is a citrate buffer and/or phosphate buffer.

3. Process according to one of the preceding claims, **characterized in that** the powder has an average particle diameter between 1 and 400 $\mu$m.

4. Process according to one of the preceding claims, **characterized in that** the polyamide used is a polyamide 11 and/or polyamide 12.

5. Process according to one of the preceding claims, **characterized in that** the polyamide has been prepared in a precipitation process or by anionic solution polymerization.

6. Process for the preparation of a polyamide powder for cosmetic applications according to one of the preceding claims, **characterized in that**, the buffer system is mixed in in the dry blend.

7. Process for the preparation of a polyamide powder for cosmetic applications according to one of the preceding claims, **characterized in that**, the buffer system is sprayed in solution onto the polyamide powder and then dried.

**Revendications**

1. Procédé de préparation d'une poudre de polyamide pour applications cosmétiques, **caractérisé en ce que**, par incorporation dans la poudre de polyamide d'un système tampon ajouté, constitué d'acides organiques et/ou naturels minéraux, et de leurs sels correspondants sous forme de bases, on ajuste le pH de la poudre de polyamide à une valeur comprise entre 4,5 et 6,5 et on utilise le système tampon en des quantités de 0,01 à 2 parties du tampon pour 100 parties du polyamide.

2. Procédé selon la revendication 1, **caractérisé en ce qu'**on utilise en tant que système tampon un tampon citrate et/ou phosphate.

3. Procédé selon l'une des revendications précédentes, **caractérisé en ce que** la poudre présente une granulométrie moyenne comprise entre 1 et 400 $\mu$m.

4. Procédé selon l'une des revendications précédentes, **caractérisé en ce qu'**on utilise en tant que polyamide un polyamide 11 et/ou un polyamide 12.

5. Procédé selon l'une des revendications précédentes, **caractérisé en ce que** le polyamide a été préparé par un procédé par précipitation ou par une polymérisation anionique en solution.

6. Procédé de préparation d'une poudre de polyamide pour applications cosmétiques selon l'une des revendications précédentes, **caractérisé en ce que** le système tampon est incorporé par mélange dans le mélange sec.

7. Procédé de préparation d'une poudre de polyamide pour applications cosmétiques selon l'une des revendications précédentes, **caractérisé en ce que** le système tampon est pulvérisé en solution sur la poudre de polyamide, puis est séché.

**EP 1 319 681 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 04050232 B
- JP 05070598 B
- JP 05025019 B
- DE 2906647 B1
- DE 2906647